# EUROPEAN PATENT APPLICATION

(11) **EP 3 603 565 A1**
(43) Date of publication of application: **05.02.2020**
(21) Application number: 19186793.6
(22) Date of filing: 17.07.2019
(51) Int. Cl.: A61C 7/08, A61C 7/36, A63B 23/03, A61F 5/56

(54) **AN ORAL STIMULATOR**

(30) Priority: 31.07.2018 IT 201800007702
(71) Applicant: Cehic, Aldo, 37135 Verona (IT)
(72) Inventor: CEHIC, Aldo, 37135 Verona (IT)
(74) Representative: Trentin, Michele

(57) **Abstract**

An oral stimulator comprising a homeoactivator **(2)** having a body **(3)** made with a substantially U-shaped shaping, a first groove **(6)** capable of receiving inside it at least one portion of the upper dental arch of a patient being provided on the upper surface **(5)** of the body, a second groove **(8)** capable of receiving inside it at least one portion of the patient's lower dental arch being provided on the lower surface **(7)** of the body. The first groove **(6)** comprises a first portion **(10)** shaped to match the teeth of the upper arch according to a desired position of such teeth, the second groove **(8)** comprising a second portion **(11)** shaped to match the teeth of the lower arch according to a desired position of such teeth.

## Description

### Definitions

Oral activators herein refer to removable appliances made of a soft material which have the characteristic of optimizing the conformation of the dental arches in the place of the typical rigid fixed and removable orthodontic appliances.

Homeoactivators refer to removable oral appliances made of a soft material which have the characteristic of being compliant with the patient's constitutive arrangement, *i.e.,* the constitution biotype referred to, in the field of homeopathy, by the terms carbonic, sulphuric and phosphoric, characterized by types of more or less pronounced curves.

Oral stimulator refers to a removable appliance which has the characteristic of stimulating at least prominently a new spatial arranging of teeth.

Soft material refers to an at least partially elastic material.

### Field of Application

The present invention generally applies to the technical fields of dentistry and physiotherapy and refers especially to the use of removable equipment to optimize a patient's bone, dental, postural, and lingual conformation.

More in detail, the present invention relates to oral stimulators addressing the alignment of the teeth and the overall reconfiguration of the oral apparatus, whose effects are also beneficial to posture.

### Background Art

In the prior art, multiple methods are used for the realignment of the teeth.

Fixed appliances, which consist of brackets made of metal or a synthetic material glued to the teeth and a metallic or synthetic wire connecting the aforementioned plates and whose tension subjects the teeth to efforts tending to arrange them according to the desired line, are known.

Removable appliances which are made of a typically hardened resinous material and are anchored to teeth by means of hooks are also known. They are activated by expansion screws and induce various types of tensions on the teeth.

Removable appliances comprised of braces made of a soft biocompatible material shaped to match the teeth according to positions other than the current ones are also known. The therapeutic strategy consists of a series of braces anchoring to the teeth and inducing a series of movements which lead them to align.

All these techniques, despite their diversity, are characterized by their direct anchoring to the teeth, thus causing an exclusive action on the teeth. Since the goal is to realign the teeth, it has been always planned to find solutions to move directly and exclusively the teeth in order to realign them.

However, it is known that, once the result is achieved, teeth tend to move independently to return to the initial configuration. Therefore, over the years, a relapse involving the majority of patients treated occurs.

It is also known that, in the search for equipment allowing to improve the health and well-being of patients, the development of multi-functional soft activators has recently been included.

Such activators were initially intended to replace, even temporarily, the use of rigid fixed or removable appliances to correct the incorrect arrangement of the teeth in the arch. However, during such uses, it was noticed that the soft activators not only achieved a postural correction of the teeth, but also gave to users a state of physical and mental well-being.

From these observations a therapeutic standard, consisting in restoring the human psycho-postural-energetic unity, has been developed.

From a strictly practical point of view, it has been observed that the biotypical constitution of the oral region (the patient's constitution biotype), with particular reference to the morphology of the bone arches and the spatial arrangements of the teeth, may be coded into three categories, *i.e.,* there are three types of dental arches: a type in which the dental arches are square, one in which the dental arches are rounded, and a type in which the dental arches are ogivally rounded.

As a result, three types of activators have been developed from these observations, each suitable for a respective biotype of the oral region. They have been called homeoactivators, where the suffix 'homeo' is related to a bioconstitutional meaning of the homeopathic type.

Moreover, a method of selecting an activator for use by a patient which allows the activator itself to achieve results with a greater degree of certainty with respect to known methods has been recently developed.

Specifically, this selection method is based on identifying with certainty which is the most suitable activator to apply to the patient in order to ensure that s/he benefits the most from it and, therefore, enjoys the greatest possible well-being.

However, such activators, also referred to as homeoactivators, are soft appliances having a space within which the teeth and a partial mandibular and maxillary bone portion are housed, and which are also characterized by the realignment of the teeth, as well as by the reshaping of the dental arches as a whole, but with far higher therapy times, lasting even months or years.

Removable appliances for improved nocturnal breathing described in document WO 2009/135210 A2, removable appliances for repositioning the teeth described in document US 2 467 432 A, removable protection appliances described in US 2010/129763 A1, and in particular dental appliances for correcting the conformation of the oral arch described in document US2003/224314 A1 are also known.

However, such appliances for the teeth alignment and protection and the overall reconfiguration of the oral apparatus do not consider the patient's constitution biotype as a characteristic for a better adaptability of the appliance itself to the oral cavities and for an increased efficacy of therapies.

### Presentation of the Invention

The object of the present invention is to at least partially overcome the drawbacks highlighted above by providing an oral stimulator which at least partially combines the spatial rearranging of teeth and their realignment in therapeutic times lower than in the equivalent prior art.

Specifically, an object of the present patent is to provide an oral stimulator which allows to reconfigure the patient's oral apparatus so that the realignment of the teeth is as physiological as possible and, as a result, is subject to a lower percentage of relapses compared to what happens in the prior art.

A further object is that the stimulation on the teeth is gradual so as to cause the least traumatic movement as possible.

Another object is that the oral stimulator dynamizes the dental structure and guide it towards a coherent spatial conformation, the most suitable one for oral functions (oral ventilation, mastication, phonetics, swallowing, suction, and taste).

A further object is that the oral stimulator allows to achieve a spatial arrangement of the teeth and of the bone matrix, including the oral and perioral soft tissues, such that the oral and perioral functions are maintained and functionally defended within the normofunctional tracks. It is indeed the new normofunction which dictates the shape of both the arches and the spatial arrangement of teeth.

In other words, an object of the present invention is to provide an oral stimulator which allows to reconfigure the oral apparatus of a patient so that it reaches an oral and perioral function, as well as an aesthetic function, suited to the patient's constitution biotype. In this sense, relapses on the teeth alignment would be limited, where not substantially eliminated.

Said objects, as well as others which will become more manifest below, are achieved by an oral stimulator according to the following claims, which are to be considered as an integral part of the present patent.

Specifically, it comprises a homeoactivator having a body typically made of a soft material with a substantially U-shaped shaping. On the upper surface of the body, there is a first groove capable of receiving inside it at least one portion of the upper dental arch of a patient. Similarly, the body also has, on the lower surface, a second groove capable of receiving inside it at least one portion of the lower dental arch of the patient.

This way, advantageously, the homeoactivator, due to its shape and its degree of elasticity, can perform its function of conforming the dental arches according to the most suitable shaping.

According to an aspect of the invention, the first groove comprises at least one first portion shaped to match the teeth of the upper arch according to a desired position of the teeth themselves. Similarly, the second groove comprises at least one second portion shaped to match the teeth of the lower arch according to a desired position of such teeth.

In other words, the oral stimulator of the invention corresponds to the coupling of a homeoactivator and a pair of braces for aligning the teeth arranged in the upper and lower grooves of the homeoactivator.

Therefore, advantageously, the oral stimulator not only moves the teeth aligning them according to a desired position, but also coordinates said movement with the bone reconfiguration of the oral arches according to the homeoactivator used.

Therefore, still advantageously, the two imposed movements enable the entire oral apparatus to be conformed according to a desired shape that is more suited to the patient.

Still advantageously, associating the movement of the teeth to the conformation of the two dental arches allows to limit, where not to eliminate, relapses since the final position of the teeth is in agreement and harmonized with the conformation of the dental arches.

Still advantageously, compared to the known simple activators, the oral stimulator of the invention allows the teeth to be realigned in much faster times.

Since the teeth realignment typically takes place by using a series of plastic templates, also referred to braces, it is clear that in order to reconfigure the oral apparatus a series of oral stimulators will be used, all referable to the coupling of a same type of homeoactivator with a series of pairs of plastic templates.

Therefore, advantageously, the reconfiguration of the oral apparatus takes place step by step and, therefore, with a series of stimulations on the teeth and on the dental arches.

The said objects are also achieved by a method of implementing an oral stimulator in accordance with the following claims.

Specifically, such method comprises the following steps:
- identifying the constitution biotype of a patient;
- selecting an oral homeoactivator in accordance with said identified biotype, said homeoactivator comprising an upper groove and a lower groove to at least partially receive the patient's upper dental arch and lower dental arch, respectively;
- identifying the initial positions of the teeth in each of the patient's dental arches;
- identifying the correct final position that the teeth in the patient's dental arches should have also based on said identified biotype;
- finding a plurality of intermediate positions between said initial position and said final position of the teeth for each of said dental arches so as to find a plurality of pairs of intermediate positions, one for the teeth of the upper dental arch and one for the teeth of the lower dental arch;
- providing a plurality of pairs of aligners, each for a respective pair of said intermediate positions;
- sequentially inserting said pairs of aligners into the respective said upper groove and lower groove of said homeoactivator so that the patient, by wearing each oral stimulator of the series of stimulators thus obtained for a predetermined period of time, can modify the mandibular and maxillary bone conformation and the position of the teeth in the dental arches according to the desired final configuration corresponding to its biotype.

Advantageously, the use of a homeoactivator allows a reconfiguration of the oral apparatus which is particularly stable and comfortable for the patient, since it can be traced back to the most suitable configuration for its physical and mental features.

Still advantageously, it follows that the final teeth alignment is also particularly stable over time not only because it is inserted in a reconfiguration also of the dental arches, but also because such reconfiguration is in accordance with the patient's biotype.

### Brief description of the drawings

Further features and advantages of the invention will become more apparent in light of the detailed description of a preferred, but not exclusive, embodiment of an oral stimulator according to the invention, illustrated by way of non-limiting example with the aid of the accompanying drawings, wherein:
- FIG. 1 shows a perspective view of an oral stimulator according to the invention;
- FIGG. 2 and 3 show details of the stimulator in FIG. 1.

### Detailed description of some preferred embodiments

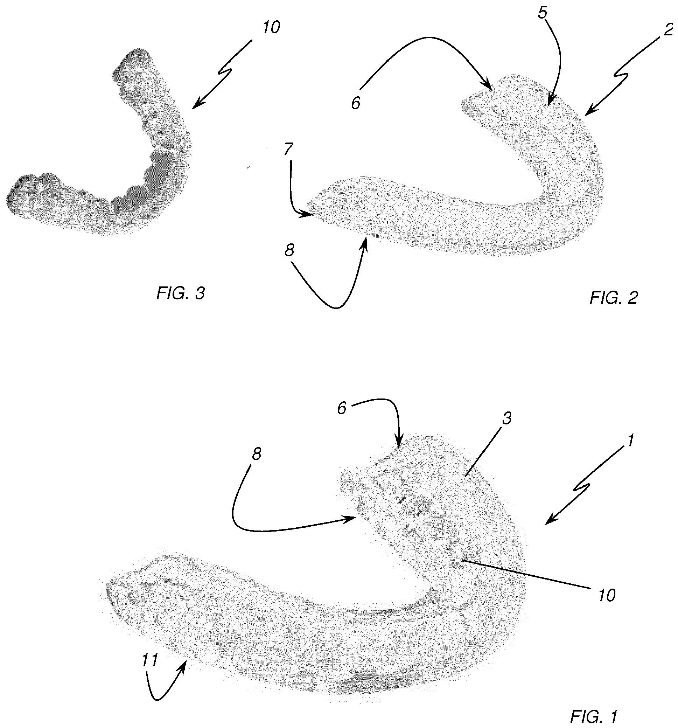

With reference to the mentioned figures, and especially to Fig. 1, an oral stimulator 1 according to the invention is described.

It is typically made of a soft material, but such detail should not be considered as limiting the present invention. However, a soft material allows to ensure an elastic behaviour. Formerly, such instruments were made of rubber, while recently materials such as silicones or thermoplastic materials are generally preferred. Neither this detail should be considered as limiting the present invention.

The oral stimulator **1** first comprises a homeoactivator **2,** also visible in the detail of Fig. 2, having a body **3** with a substantially U-shaped shaping. Such shaping, as will be seen better later, may be more or less rounded in accordance with the patient's biotype.

On the upper surface **5** of the body **3,** as is known, there is a first groove **6** capable of receiving inside it the patient's upper dental arch. Similarly, on the lower surface **7** of the body **3,** there is a second groove **8** capable of receiving inside it the patient's lower dental arch.

In case of known activators, the first and second grooves have a rather wide bottom so as not to affect the teeth as a whole, but only the overall dental arch. In this sense, the possible teeth realignment is a secondary phenomenon which occurs over months or years of treatment.

By contrast, in case of the oral stimulator **1** of the invention, the first groove **6** comprises a first portion **10,** which can also be seen in the detail of Fig. 3, shaped to match the teeth of the upper arch according to a desired position of such teeth. Similarly, the second groove **8** comprises a second portion **11** shaped to match the teeth of the lower arch according to a desired position.

In other words, the first and second grooves **6** and **8** are not only shaped based on the shape that the respective dental arches must take, but also on the position that the teeth of such arches must assume.

Therefore, advantageously, the oral stimulator **1** of the invention combines the action of reconfiguration or maintenance of the shaping of the dental arches with the action of teeth realignment.

Such actions, by the way, are implicitly and advantageously coordinated, thus ensuring an optimal result and favouring the stability of the final position of arches and teeth.

It may already been anticipated, as will be seen in detail below, that, since typically the final position of realignment of the teeth is distinctively different from the initial position of the same teeth, such action of realignment will develop in multiple steps, each using the same type of Homeoactivator **2,** but having first and second portions **10** and **11** differing from a step to another according to a series of predetermined movements.

Therefore, advantageously, the reconfiguration of the oral apparatus is carried out by a series of steps and with limited efforts imposed both on the teeth and on the bone matrix of the dental arches.

According to the embodiment being described, the first and second portions **10** and **11** are made integrally with the homeoactivator **2.** In other words, the oral stimulator **1** is made of a single body. However, this should not be considered as limiting the invention. According to an alternative embodiment, actually, the first and second portions are respectively formed as a first additional body and a second additional body stably coupled into the first groove and into the second groove of the homeoactivator, respectively.

According to an aspect of the invention, the oral stimulator **1** also comprises a shaped lingual portion (not shown in the figures) intended to be arranged in the sublingual area of the patient to keep the tongue arranged according to a position of improved functional and energetic comfort.

It is known, indeed, that the tongue is not always in a correct position, and that there are positions of the tongue which are relaxing for the whole organism and positions which dysfunctionally stimulate the organism. In this sense, the lingual portion allows the tongue to be kept in the best position during treatment with the stimulator **1** in order to let it get used to staying in that position. As a result, the patient's well-being is advantageously increased, and the reconfiguration of the oral apparatus is improved.

From the foregoing, it is clear that the object of the invention is also the method of implementing the oral stimulator **1** hitherto described.

According to this method, there are steps for selecting the homeoactivator **2.** In this sense, there is a step of identifying the patient's biotype.

The choice of the homeoactivator **2** should not be arbitrary or theoretical, but should be based on physical parameters which allow to identify the correct biotype of the patient's oral region so that the effect of the homeoactivator **2** is positive and maximized.

To achieve this end, according to a non-limiting aspect of the invention, the method comprises a first step of identifying the patient's biotype. Such step is typically carried out through a homeopathic study of the patient, but such characteristic should not be considered as limiting the invention.

Moreover, this step can be carried out in different ways, even though it typically takes place through the observation of particular features of the patient. In homeopathy, indeed, it is known that the sulphuric biotype has a normal constitution, medium and harmonious stature, rectangular and well-proportioned face in its parts, square and solid teeth, infrequent caries, circular arches, rounded hard palate, perfect occlusion, hands with similar lengths between the fingers and the palm, lively reactions, sensual excesses, and is cold enthusiast, self-controlled.

The carbonic biotype is typically short, obesity-prone, has a squat appearance, square face, square teeth, tendency to caries, wide arches, flat hard palate, perfect occlusion, it has squat hands with fingers shorter than the palm, big fingertips, slow and not explosive reactions. They are cold subjects, fear the sun, love peace and order, are passive and methodical.

By contrast, the phosphoric biotype is taller than average, with reduced weight compared to height, a roughly triangular and elongated face (dolichocephaly) with a high forehead, elegant hands with fingers longer than the palm, rectangular teeth, which are not robust and prone to caries, anteroposteriorly elongated arches, deep hard palate, imperfect occlusion. The reactions are typically lively, but brief due to a sense of general asthenia. They need fresh air, are easy to tire, have no patience.

For the descriptions given, it is clear that a patient hardly may be totally identified in a precise biotype, but has peculiarities typically of two or more different biotypes, that is why more than one homeoactivator models may apparently be used. In addition, it is clear that each patient has different sizes of the dental arches. In this sense, it is necessary to measure them. However, by identifying the biotype, models of homeoactivators **2** that are not suitable for the patient have been eliminated with certainty, leaving a subset of all those available.

According to another aspect of the invention, then there is a step of checking the postural balance of the patient which uses the selected homeoactivators **2.** In other words, the patient's postural balance is checked while s/he is wearing each of the homeoactivators **2** selected with the previous steps. Specifically, such checking is carried out using appropriate means of measuring postural balance.

After that, the most suitable homeoactivator **2** corresponding to the one with which the detected postural balance is better is selected.

It is also necessary to detect the initial arrangement of the patient's teeth. Typically, this is made by making dental impressions of the arches and scanning them directly or by scanning the plaster models obtained therefrom, but this aspect must not be considered as limiting the invention. Besides, according to some alternative embodiments, first this is made through relaxation operations of the muscular apparatus of the mouth, for example by means of TENS, but also such detail must not be considered as limiting the invention.

Once the initial position of the teeth has been acquired, their final position, *i.e.,* the position aligned and shaped according to the dental arches in accordance with the patient's biotype, is determined.

Since the initial and final positions of the teeth of the two arches can be very different, the method of the invention provides for a step of determining a plurality of intermediate positions for each of the dental arches so as to identify a plurality of pairs of intermediate positions, one for the teeth of the upper dental arch and one for the teeth of the lower dental arch.

The number of intermediate positions to be identified is personal and follows relevant individual paths, whereby there is no pre-established number to reach the orthodontic optimum. Each case has its own strategy and its own number of intermediate positions.

A series of oral stimulators **1,** made up of the integration of homeoactivators **2** of the chosen type with pairs of plastic templates inserted into the upper and lower grooves of the homeoactivator **2,** is thus obtained. Such series of oral stimulators **1** identifies, indeed, the path of teeth realignment within the more general reconfiguration of the oral apparatus. This implementation step may be carried out with several methodologies, such as, for example, 3D printing.

After that, the oral stimulator **1** is finally implemented.

According to the embodiment being described, for each pair of aligners, they are then inserted into the respective grooves **6, 8** of the homeoactivator **2,** after using adhesives or any other means capable of favouring their stable coupling.

The series of assemblies thus obtained is used to provide a series of casts made of plaster, or of another material, of the resulting dental arches. Such casts will then be used to implement the series of oral stimulators **1** to be used in the treatment of the patient. The sequential use of such oral stimulators **1,** each for a predetermined period of time, allows to modify both the mandibular and maxillary conformation and the position of the teeth in the dental arches according to the desired final configuration corresponding to the patient's biotype.

Such conformation is therefore advantageously stable, since it is the patient's own one, according to his psycho-physical features.

Still advantageously, this allows the patient to achieve a conformation of the oral apparatus which is comfortable and capable of favouring and stimulating his psycho-physical well-being.

Although it has been said that the homeoactivator - aligners assembly is used to make casts, this aspect must not be considered as limiting the invention. According to some alternative embodiments, indeed, the coupling between the homeoactivator **2** and the aligners is made in a particularly stable manner so as to allow such assembly to form the oral stimulator **1** immediately usable by the patient.

According to another aspect of the invention, after the step of stable coupling the aligners into the grooves **6** and **8** and before the step of providing the casts, there are some additional steps.

Specifically, there is a step of inserting the aligners - homeoactivator coupling into the patient's mouth. It is then made sure that the patient arranges the tongue so that its tip is in contact with the interincisal papilla located in the palate between the two upper central incisors. At this point, a biocompatible plastic material is injected into the mouth to fill the lower area of the patient's mouth below the floor of the tongue. When the material hardens, it is coupled by contact with the homeoactivator **2** and forms the lingual portion described above. Once the whole has been extracted from the patient's mouth, the overall assembly is therefore made up of the homeoactivator **2,** the aligners and the lingual portion.

In light of the foregoing, it is understood that the oral stimulator of the invention and the method for implementing it achieve all the prefixed objects.

Specifically, the resulting oral stimulator combines the spatial rearranging of the jaw and the mandible and the teeth realignment. Moreover, it also combines postural education of the tongue.

Ultimately, the oral stimulator of the invention allows to reconfigure the patient's oral apparatus so that the teeth realignment remains permanent or undergoes, after treatment, anything but imperceptible movements, since it is harmonized with the conformation of the dental arches. This configuration is particularly stable, since it is in accordance with the patient's biotype. It also takes place in faster treatment times than the times required when known activators are used.

The object of obtaining an oral stimulator which dynamizes the dental structure and guide it towards a coherent spatial conformation, the most suitable one for oral functions (oral ventilation, mastication, phonetics, swallowing, suction, and taste) is definitely achieved.

Specifically, the oral stimulator of the invention allows to reconfigure the oral apparatus of a patient so that it reaches an oral and perioral function, as well as an aesthetic function, suited to the patient's constitution biotype. In this sense, relapses on the teeth alignment are limited, where not substantially eliminated.

The invention might be subject to many changes and variants, which are all included in the appended claims. All the details may be replaced by other technically equivalent elements, and the materials may be different depending on the needs, without departing from the protection scope of the invention defined by the appended claims.

## Claims

1. An oral stimulator comprising a homeoactivator (**2**) having a body (**3**) made with a substantially U-shaped shaping, a first groove (**6**) capable of receiving inside it at least one portion of the upper dental arch of a patient being provided on the upper surface (**5**) of said body, a second groove (**8**) capable of receiving inside it at least one portion of the patient's lower dental arch being provided on the lower surface (**7**) of said body, **characterized in that** said first groove (**6**) comprises at least one first portion (**10**) shaped to match the teeth of the upper arch according to a desired position of such teeth, said second groove (**8**) comprising at least one second portion (**11**) shaped to match the teeth of the lower arch according to a desired position of such teeth.

2. The oral stimulator according to claim 1, **characterized in that** said first and second portions (**10, 11**) are formed integrally with said homeoactivator (**2**).

3. The oral stimulator according to claim 1, **characterized in that** said first and second portions are respectively formed as a first additional body and a second additional body stably coupled into said first groove and into said second groove, respectively.

4. The oral stimulator according to any one of the preceding claims, **characterized in** comprising a shaped lingual portion intended to be arranged in the sublingual area to keep the patient's tongue in a position of improved functional and energetic comfort.

5. A method of implementing an oral stimulator (**1**) comprising the following steps:
- identifying the constitution biotype of a patient;
- selecting a homeoactivator (**2**) in accordance with said identified biotype, said homeoactivator (**2**) comprising an upper groove (**6**) and a lower groove (**8**) to at least partially receive the patient's upper dental arch and lower dental arch, respectively;
- identifying the initial positions of the teeth in each of the patient's dental arches;
- identifying the correct final position that the teeth in the patient's dental arches should have also based on said identified biotype;
- finding a plurality of intermediate positions between said initial position and said final position of the teeth for each of said dental arches so as to find a plurality of pairs of intermediate positions, one for the teeth of the upper dental arch and one for the teeth of the lower dental arch;
- providing a plurality of pairs of aligners, each for a respective pair of said intermediate positions;
- sequentially inserting said plurality of pairs of aligners into the respective said upper groove (**6**) and lower groove (**8**) of said homeoactivator (**2**) so that the patient, by wearing each oral stimulator (**1**) of the series of stimulators thus obtained for a predetermined period of time, can modify the mandibular and maxillary conformation and the position of the teeth in the dental arches according to the desired final configuration corresponding to its biotype.

6. The method according to claim 5, **wherein** after said insertion of one of said pairs of aligners in said respective grooves (**6, 8**) of said homeoactivator, there are the following steps:
- stably coupling said aligners into said grooves (**6, 8**);
- making a plaster cast of the dental arches suitable for using said coupling between said pair of aligners and said homeoactivator (**2**);
- using said plaster cast to make said oral stimulator (**1**) of a series of said oral stimulators (**1**) corresponding to said series of intermediate positions of the teeth.

7. The method according to claim 5, **wherein** after said insertion of one of said pairs of aligners into said respective grooves of said homeoactivator, there is a step of stably coupling said aligners into said grooves to make said oral stimulator thus obtained immediately usable by the patient.
